# EUROPEAN PATENT APPLICATION

(11) **EP 4 269 387 A1**
(43) Date of publication of application: **01.11.2023**
(21) Application number: 21908277.3
(22) Date of filing: 26.01.2021
(51) Int. Cl.: C07D 207/38

(54) **PYRROLINONE COMPOUND AND SYNTHESIS METHOD THEREFOR**

(30) Priority: 22.12.2020 CN 202011526131
(71) Applicant: Poseidon Pharmaceutical Co., Ltd., Hong Kong 999077 (CN); Wuhan Dapeng Pharmaceutical Co., Ltd., Wuhan, Hubei 430014 (CN)
(72) Inventor: CHEN, Fapu, Wuhan, Hubei 430014 (CN); SHI, Yuxin, Wuhan, Hubei 430014 (CN); CHEN, Fakai, Wuhan, Hubei 430014 (CN)
(74) Representative: Scholl, Matthias
(86) International application number: PCT/CN2021/073780
(87) International publication number: WO 2022/134259

(57) **Abstract**

The present invention provides a pyrrolinone compound of which the structural formula is as shown in formula 1. R₁ is selected from one of: C₁-C₅ alkoxy groups, a benzyloxy group, C₁-C₅ alkyl groups, and a phenyl group; R₂ and R₃ are each independently selected from one of: hydrogen, C₁-C₅ alkyl groups, C₁-C₅ alkoxy groups, C₁-C₅ alkylthio groups, C₁-C₅ alkylsulphinyl groups, C₁-C₅ alkylsulfonyl groups, different substituted phenyl groups, different substituted phenoxy groups, different substituted phenylthio groups, different substituted phenylsulphinyl groups, and different substituted phenylsulfonyl groups; and n₁ and n₂ are integers selected from 1 to 5.The compound can be used as a pharmaceutical intermediate. The present invention also provides a synthesis method for the pyrrolinone compound, which is suitable for industrialization, low in cost, and mild in synthesis conditions.

## Description

The disclosure relates to a pyrrolinone compound and a method for preparing the same.

Pyrrolidone is an important structural segment of glimepiride, bilirubin, biliverdin, phycocyanin and other drugs and food additives.

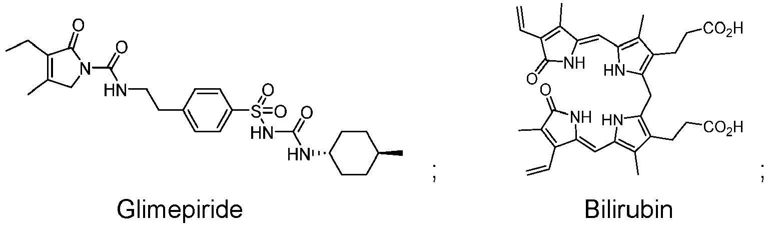

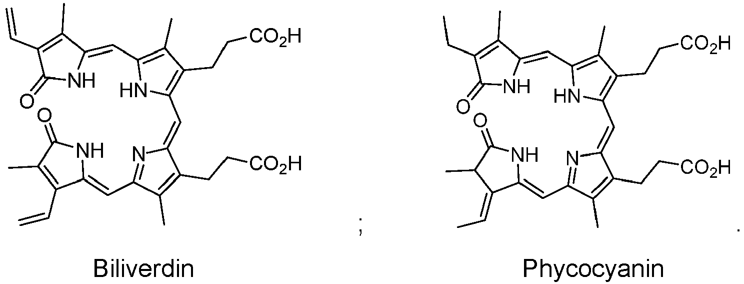

Conventional methods for preparing a pyrrolinone compound includes the following means:

### 1. With 1-tosylpyrrole as starting material

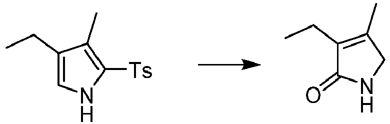

The method (Monatsh. Chem., 2014, 145, 5, 775-789) uses Tosyl pyrrole as the raw material, through bromination, hydrolysis, reduction and other steps to obtain a pyrrolidone compound. During the hydrolysis process, a large amount of trifluoroacetic acid is consumed, leading to environmental pollution. Sodium borohydride is used for reduction, which is costly.

### 2. With pyrrolamide as starting material

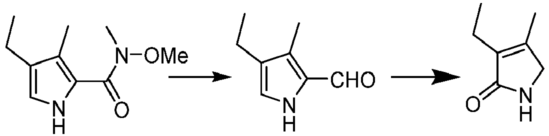

The method (J. Org. Chem.2006,71, 6678-6681) uses pyrrolamide as the raw material, which is reduced by lithium aluminum hydride to form pyrrolaldehyde, and then oxidized to produce pyrrolinone. The lithium aluminum hydride used in this method is expensive and dangerous, making it unsuitable for industrial production.

### 3. With ethyl acetoacetate as starting material

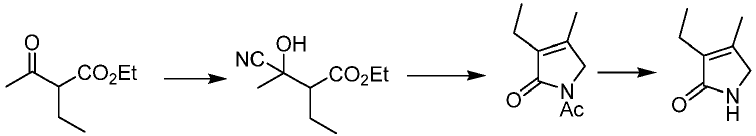

The method (Tetrahedron Lett., 2003, 44, 4853 - 4855) uses ethyl acetoacetate as the starting material, followed by addition of sodium cyanide, catalytic hydrogenation, and hydrolysis to obtain a pyrrolidone compound. The raw material used in this method, sodium cyanide, is highly toxic. Catalytic hydrogenation requires high pressure, with a yield of only 15%, which limits the industrial production.

### 4. With acrylamide as starting material

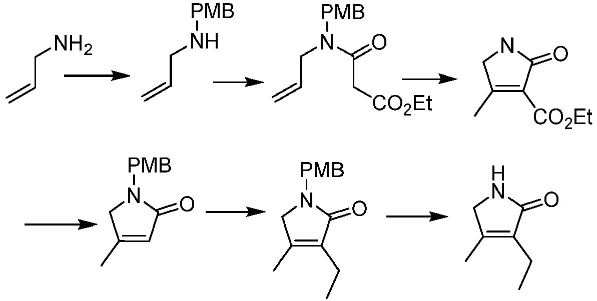

Compared with the above methods, the method (Synthesis, 2015, 47, 955-960) involves no reagents such as lithium aluminum hydride, sodium cyanide, trifluoroacetic acid, and catalytic hydrogenation. However, reagents such as sodium borohydride and expensive palladium chloride are still required.

To solve the aforesaid problems, the disclosure provides a pyrrolinone compound and a method for preparing the same.

The pyrrolinone compound represented by formula 1 is as follows: 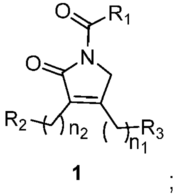 where, R₁ is selected from one of C₁-C₅ alkoxy, benzyloxy, C₁-C₅ alkyl, or a phenyl group; R₂, R₃ at each occurrence independently represent hydrogen, C₁-C₅ alkyl, C₁-C₅ alkoxy, C₁-C₅ alkylthio, C₁-C₅ alkane sulfinyl, C₁-C₅ alkane sulfonyl, a substituted phenyl, a substituted phenoxy, a substituted phenylthio, a substituted phenylene sulfinyl, or a substituted benzene sulfonyl; and n₁, n₂ at each occurrence independently are an integer from 1 to 5. The term "substituted" refers to substitutions at various positions, such as meta substitution, ortho substitution, para substitution, and further refers to different substituents, such as C₁-C₅ alkyl substitution, or halogen substitution.

In a class of this embodiment, R₃ is hydrogen, and n₁ is 1.

In a class of this embodiment, n₂ is 2.

In a class of this embodiment, the abovementioned compound has one of following formulas:

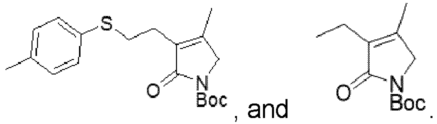

The disclosure also provides a method for preparing the abovementioned compound comprising applying a compound represented by formula 6 to synthesize the compound of formula 1:

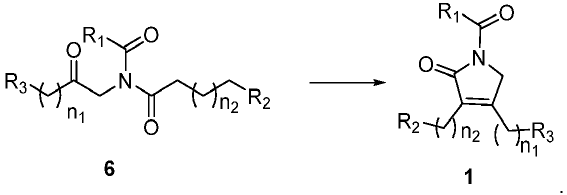

In a class of this embodiment, a starting compound is dissolved in a solvent and catalyzed by a base to yield the compound represented by formula 6.

In a class of this embodiment, the solvent is tetrahydrofuran, methanol, ethanol, water, acetone, dimethyl formamide (DMF), dimethylsulfoxide (DMSO), or a mixture thereof; and the base is sodium hydroxide, potassium hydroxide, sodium methoxide, sodium ethanol, potassium tert butanol, sodium tert butanol, 1,8-diazabicyclo (5.4.0) undec-7-ene (DBU), dimethylaminopyridine, or a mixture thereof.

In a class of this embodiment, the compound represented by formula 6 is prepared by a compound represented by formula 5 as follows:

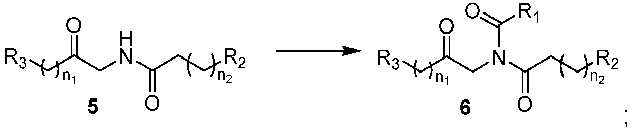

R₁ is selected from one of C₁-C₅ alkoxy, benzyloxy, C₁-C₅ alkyl, or a phenyl group; R₂, R₃ at each occurrence independently represent hydrogen, C₁-C₅ alkyl, C₁-C₅ alkoxy, C₁-C₅ alkylthio, C₁-C₅ alkane sulfinyl, C₁-C₅ alkane sulfonyl, a substituted phenyl, a substituted phenoxy, a substituted phenylthio, a substituted phenylene sulfinyl, or a substituted benzene sulfonyl; and n₁, n₂ at each occurrence independently are an integer from 1 to 5. The term "substituted" refers to substitutions at various positions, such as meta substitution, ortho substitution, para substitution, and further refers to different substituents, such as C₁-C₅ alkyl substitution, or halogen substitution.

In a class of this embodiment, the compound represented by formula 5 is dissolved in a solvent and reacts with an anhydride or acyl chloride in the presence of a base to yield the compound represented by formula 6.

In a class of this embodiment, the base is triethylamine, pyridine, dimethylaminopyridine, diisopropylethylamine, sodium carbonate, potassium carbonate, sodium bicarbonate, or a mixture thereof; the solvent is dichloromethane, chloroform, tetrahydrofuran, acetone, and ethyl acetate, or a mixture thereof.

In a class of this embodiment, a molar ratio of the compound represented by formula 5 to the acyl chloride or anhydride to the base is 1: (0.8-2): (0.8-2).

In a class of this embodiment, the compound represented by formula 6 is prepared by the compound represented by formula 5 under a temperature of 0-30°C.

In a class of this embodiment, the compound represented by formula 5 is prepared by a compound represented by formula 4:

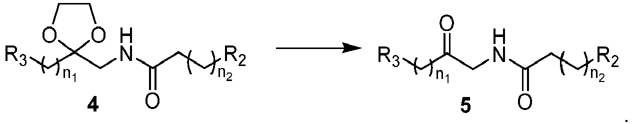

In a class of this embodiment, the compound represented by formula 4 is dissolved in a solvent and converted into the compound represented by formula 5 through a deprotection reaction in the presence of an acidity regulator.

In a class of this embodiment, the acidity regulator is hydrochloric acid, sulfuric acid, formic acid, acetic acid, or a mixture thereof; and the solvent is dichloromethane, chloroform, tetrahydrofuran, acetone, and ethyl acetate, or a mixture thereof.

In a class of this embodiment, a molar ratio of the compound represented by formula 4 to the acidity regulator is between 1: 0.1 and 1:1.

In a class of this embodiment, the compound represented by formula 5 is prepared by the compound represented by formula 4 under a temperature of 20-30 °C.

In a class of this embodiment, the compound represented by formula 4 is prepared by reaction of a compound represented by formula 2 and a compound represented by formula 3 as follows:

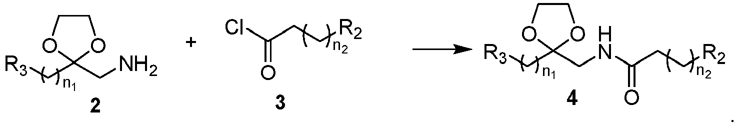

In a class of this embodiment, the compound represented by formula 2 and the compound represented by formula 3 are dissolved in a solvent and react with each other in the presence of a base to yield the compound represented by formula 4.

In a class of this embodiment, the solvent is dichloromethane, chloroform, tetrahydrofuran, acetone, and ethyl acetate, or a mixture thereof, and the base is triethylamine, pyridine, dimethylaminopyridine, diisopropylethylamine, sodium carbonate, potassium carbonate, sodium bicarbonate, or a mixture thereof.

In a class of this embodiment, a molar ratio of the compound represented by formula 2 to the compound represented by formula 3 to the base is 1: (0.8-2): (0.8-2).

In a class of this embodiment, the compound represented by formula 4 is prepared by reaction of the compound represented by formula 2 and the compound represented by formula 3 under a temperature of 15-30°C.

The following advantages are associated with the pyrrolinone compound and the method for preparing the same of the disclosure:
1. The pyrrolidone compound of the disclosure can be used as a pharmaceutical intermediate for preparing phycocyanin and glimepiride under mild conditions, with a higher yield than conventional methods.
2. The synthesis method for pyrrolidone compound of the disclosure is more suitable for industrialization, lower cost, and more mild synthesis conditions than conventional methods.

To further illustrate the disclosure, embodiments detailing a pyrrolinone compound and a method for preparing the same are described below. It should be noted that the following embodiments are intended to describe and not to limit the disclosure.

In the disclosure, NMR was measured using Bruker-AMX400 nuclear magnetic resonance instrument; ESI-MS was measured using Finnigan-MAT-95 mass spectrometer; All reagents are analytical pure (National Pharmaceutical Reagent Company). In the following examples, 2,2-dimethoxypropylamine is prepared according to the literature Eur. J. Med. Chem., 1995, 30, 931-942 method preparation; 4-p-Toluenesulfobutyryl chloride is prepared according to the method described in literature MedChemComm, 2017, 8, 1268-1274.

### Example 1

### Synthesis of Compound 4a: N- (2,2-dimethoxypropyl) -4-p-toluenesulfobutanamide

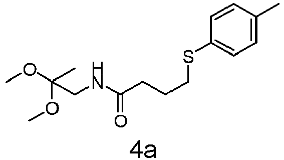

6.80 g of 2,2-dimethoxypropylamine (57.1 mmol) were weighed and dissolved in 20 mL of dichloromethane. Then, 6.10 g of triethylamine (60.4 mmol) was added to the resulting mixture and the mixture was cooled to 0°C. Thereafter, 13.03 g (57.1 mmol) of 4-p-toluenesulfobutyryl chloride dissolved in 20 mL of dichloromethane was slowly added to the mixture, and the temperature was controlled below 5°C. The mixture was stirred at 20-30°C for 10 hours and filtered to obtain N- (2,2-dimethoxypropyl) -4-p-toluenesulfobutanamide. The filter cake was washed with dichloromethane, and the filtrate was used for next reaction.

### Example 2

### Synthesis of Compound 4b: N-(2,2-dimethoxypropyl) butanamide

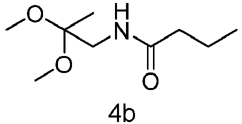

2,2-dimethoxypropylamine (55.8 mmol) was dissolved in 20 mL of tetrahydrofuran. Then, potassium carbonate (63.7 mmol) was added to the resulting mixture and the mixture was cooled to 0°C. Thereafter, 5.97 g (55.8 mmol) of butyryl chloride dissolved in 20 mL of tetrahydrofuran was slowly added to the mixture, and the temperature was controlled below 5°C. The mixture was stirred at 15-25°C for 10 hours and filtered to obtain N-(2,2-dimethoxypropyl) butanamide. The filter cake was washed with dichloromethane, and the filtrate was used for next reaction.

### Example 3

### Synthesis of Compound 5a: N-acetone-4-p-toluenesulfobutylamide

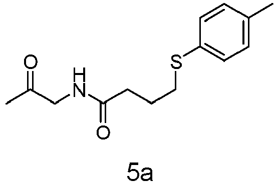

8.0 g (10.8 mmol) of 5% dilute hydrochloric acid was added to a dichloromethane solution containing the compound 4a obtained in Example 1. The mixture was stirred at 15-35°C for 4 hours and rested. The separated dichloromethane layer was collected, washed with saturated salt water, water, dried over anhydrous sodium sulfate, filtered, concentrated, to yield 10.20 g of a white solid, that is, N-acetone-4-p-toluenesulfobutylamide. The two-step yield (the total yield of the two-step reaction in Example 1 and Example 3) is 96%. N-acetone-4-p-toluenesulfobutylamide: ¹H NMR (400 MHz, CDCl₃): δ 1.92-1.97 (m, 2H), 2.20 (s, 3H), 2.31 (s, 3H), 2.39 (t, 6.8 Hz, 2H), 2.93 (t, J = 6.8 Hz, 2H), 4.14 (d, J = 4.0 Hz, 2H), 6.22 (s, 1H), 7.09 (d, J = 4.0 Hz, 2H), 7.26 (d, J = 8.0 Hz, 2H); ESI-Mass: 266.0[M+H]⁺.

### Example 4

### Synthesis of Compound 5b: N-acetone-4-butylamide

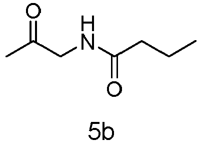

8.0 g of 5% dilute hydrochloric acid was added to a dichloromethane solution containing the compound 4b obtained in Example 2. The mixture was stirred at 15-35°C for 4 hours and rested. The separated dichloromethane layer was collected, washed with saturated salt water, water, dried over anhydrous sodium sulfate, filtered, concentrated, to yield a white solid, that is, N-acetone-4-butylamide. The two-step yield (the total yield of the two-step reaction in Example 1 and Example 3) is 92%. N-acetone-4-butylamide: ¹H NMR (400 MHz, CDCl₃): δ0.99 (t, J = 7.0 Hz, 3H), 1.64-1.70 (m, 2H), 2.21 (s, 3H), 2.34 (t, J = 6.8 Hz, 2H), 4.16 (s, 2H); ESI-Mass: 144.18[M+H]⁺.

### Example 5

### Synthesis of Compound 6a: N-acetonyl-N-tert-butyloxycarbonyl-4-p-toluenesulfobutylamide

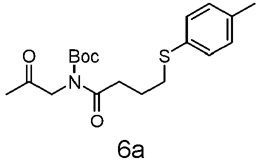

8 g of the compound 5a was dissolved in 50 mL of dichloromethane, and then 8.0 g of dimethylaminopyridine (DMAP) was added. The resulting mixture was cooled to 0°C, and 13.2 g of Boc₂O dissolved in 90 mL of dichloromethane was added. The temperature was controlled to not exceed 5°C. The mixture was stirred at 15-30°C for 10 hours and poured into 100 mL of ice water. The aqueous phase was acidified with dilute hydrochloric acid to pH 3. The organic layer was collected, washed with saturated sodium bicarbonate, salt water, dried over anhydrous sodium sulfate, filtered, and concentrated, to yield 10.81 g of light yellow liquid, which was N-acetonyl-N-tert-butyloxycarbonyl-4-p-toluenesulfobutylamide, with a yield of 99%. ¹H NMR (400 MHz, CDCl₃): δ 1.47 (s, 9H), 1.93-1.97 (m, 2H), 2.14 (s, 3H), 2.30 (s, 3H), 2.93 (t, J = 6.0 Hz, 2H),3.08 (t, J =7.2 Hz, 2H), 4.49 (s, 2H), 7.08 (d, J = 8.2 Hz, 2H), 7.25 (d, J=8.2 Hz, 2H); ESI-Mass: 387.97 [M+Na]⁺.

### Example 6

### Synthesis of Compound 6b: N-acetone-N-tert butoxycarbonyl butylamide

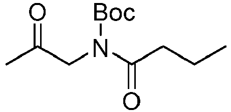

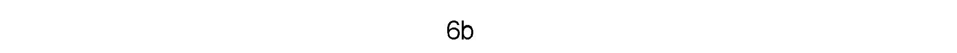

8 g of the compound 5b was dissolved in 50 mL of dichloromethane, and then 8.0 g of DMAP was added. The resulting mixture was cooled to 0°C, and 13.2 g of Boc₂O dissolved in 90 mL of dichloromethane was added. The temperature was controlled to not exceed 5°C. The mixture was stirred at 15-35°C for 10 hours and poured into 100 mL of ice water. The aqueous phase was acidified with dilute hydrochloric acid to pH 3. The organic layer was collected, washed with saturated sodium bicarbonate, salt water, dried over anhydrous sodium sulfate, filtered, and concentrated, to yield a light yellow oily product, which was N-acetone-N-tert butoxycarbonyl butylamide, with a yield of 97%. ¹H NMR (400 MHz, CDCl₃): δ 1.00 (t, J = 7.2 Hz, 3H), 1.48 (s, 9H), 1.76-1.78 (m, 2H), 2.23 (s, 3H), 2.95 (m, 2H), 4.45 (s, 2H), ESI-Mass: 244.22 [M+H]⁺.

### Example 7

### Synthesis of Compound 1a: N-tert butoxycarbonyl-3- (2-p-tolylthioethyl) -4-methyl-1H-2 (5H) pyrrolidone

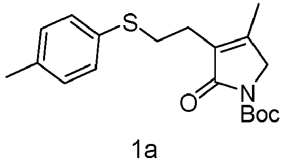

6.80 g of the compound 6a was dissolved in 50 mL of dimethylsulfoxide (DMSO) containing 3.0 g of sodium hydroxide, stirred at 15-35°C for 30 minutes. The resulting mixture was poured into 100 mL of ice water, extracted with ethyl acetate, washed with dilute hydrochloric acid to neutral, washed with saturated salt water, dried with anhydrous sodium sulfate, filtered, concentrated, and recrystallized with ethanol to obtain 6.0 g of an earthy yellow solid with a yield of 84%. ¹H NMR (400 MHz, CDCl₃): δ 1.55 (s, 9H), 1.95 (s, 3H), 2.30 (s, 3H), 2.57 (t, J = 7.2 Hz, 2H), 3.11 (t, J = 7.2 Hz, 2H), 4.03 (s, 2H), 7.08 (d, J = 8.0 Hz, 2H), 7.25(d, J = 8.0 Hz, 2H); ESI-Mass: 717.14 [2M+Na]⁺.

### Example 8

### Synthesis of Compound 1b: N-tert butoxycarbonyl-3-ethyl-4-methyl-1H-2 (5H) pyrrolidone

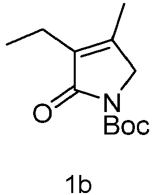

6.80 g of the compound 6b was dissolved in 50 mL of DMSO containing 3.0 g of sodium hydroxide, stirred at 15-35°C for 30 minutes. The resulting mixture was poured into 100 mL of ice water, extracted with ethyl acetate, washed with dilute hydrochloric acid to neutral, washed with saturated salt water, dried with anhydrous sodium sulfate, filtered, concentrated, and recrystallized with ethanol to obtain a colorless oily product, that is, N-tert butoxycarbonyl-3-ethyl-4-methyl-1H-2 (5H) pyrrolidone, with a yield of 77%. 1H NMR (400 MHz, CDCl₃): δ 1.10 (t, J = 7.0 Hz, 3 H), 1.49 (s, 9 H), 2.01 (s, 3 H), 2.32 (q, J = 7.4 Hz, 2 H), 4.20 (s, 2 H), ESI-Mass: 226.22 [M+1]⁺.

### Example 9

### Synthesis of an intermediate of phycocyanin with Compound 1a:

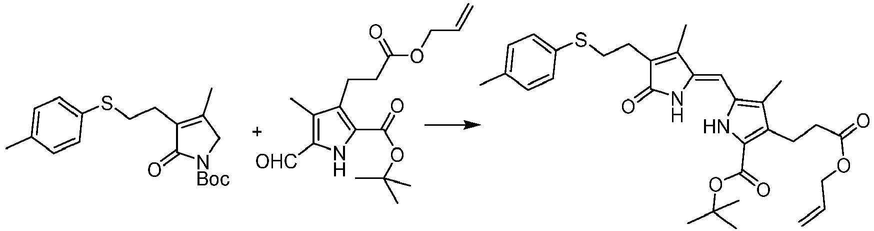

3.47 g of the compound shown in formula 1a and 3.21 g of 5-formyl-4-methyl-3-allyloxycarbonyl-ethyl-2 tert-butyl pyrrolite were mixed and dissolved in 30 mL of toluene. 5.0 g of DBU was added to the resulting mixture, heated and refluxed for 2 hours, and vacuum distillation was performed to remove the solvent. The residue was recrystallized with 20 mL of methanol to obtain 4.12 g of a yellow solid with a yield of 75%. The spectral data are the same as Synlet 1999, S1, 901-904.

### Example 10

### Synthesis of an intermediate of glimepiride with Compound 1b:

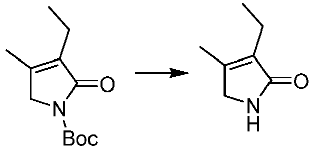

1.12 g of the compound shown in formula 1b was dissolved in 10 mL of ethyl acetate, cooled to 0°C, and 10 mL of 4 N hydrochloric acid dissolved in 15 mL of dioxane was slowly added to the resulting mixture. The temperature was slowly raised to 15-35°C, and the mixture was stirred for 2 hours, extracted with ethyl acetate, washed with saturated salt water, dried with anhydrous sodium sulfate, and separated through column chromatography to obtain 0.46 g of a white solid, with a yield of 75%. Spectral data is the same as Synthesis, 2015; 47, 955-960.

It will be obvious to those skilled in the art that changes and modifications may be made, and therefore, the aim in the appended claims is to cover all such changes and modifications.

## Claims

1. A pyrrolinone compound represented by formula 1: 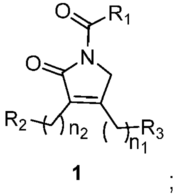 wherein:
R₁ is selected from one of C₁-C₅ alkoxy, benzyloxy, C₁-C₅ alkyl, or a phenyl group; R₂, R₃ at each occurrence independently represent hydrogen, C₁-C₅ alkyl, C₁-C₅ alkoxy, C₁-C₅ alkylthio, C₁-C₅ alkane sulfinyl, C₁-C₅ alkane sulfonyl, a substituted phenyl, a substituted phenoxy, a substituted phenylthio, a substituted phenylene sulfinyl, or a substituted benzene sulfonyl; and n₁, n₂ at each occurrence independently are an integer from 1 to 5.

2. The compound of claim 1, wherein R₃ is hydrogen, and n₁ is 1.

3. The compound of claim 2, wherein n₂ is 2.

4. The compound of claim 3, having one of following formulas:

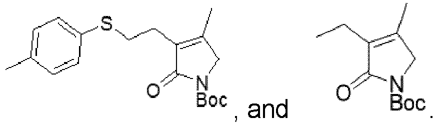

5. A method for preparing the compound of claim 1, comprising applying a compound represented by formula 6 to synthesize the compound of formula 1:

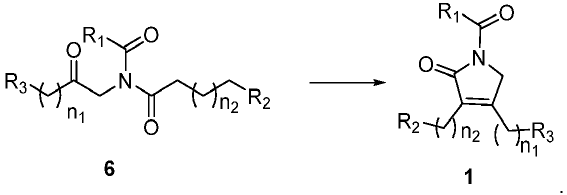

6. The method of claim 5, wherein a starting compound is dissolved in a solvent and catalyzed by a base to yield the compound represented by formula 6.

7. The method of claim 6, wherein the solvent is tetrahydrofuran, methanol, ethanol, water, acetone, dimethyl formamide (DMF), dimethylsulfoxide (DMSO), or a mixture thereof; and the base is sodium hydroxide, potassium hydroxide, sodium methoxide, sodium ethanol, potassium tert butanol, sodium tert butanol, 1,8-diazabicyclo (5.4.0) undec-7-ene (DBU), dimethylaminopyridine, or a mixture thereof.

8. The method of claim 5, wherein the compound represented by formula 6 is prepared by a compound represented by formula 5 as follows: 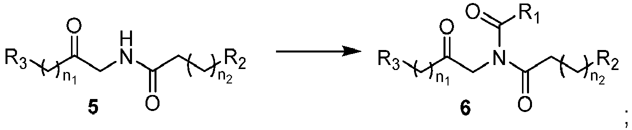 R₁ is selected from one of C₁-C₅ alkoxy, benzyloxy, C₁-C₅ alkyl, or a phenyl group; R₂, R₃ at each occurrence independently represent hydrogen, C₁-C₅ alkyl, C₁-C₅ alkoxy, C₁-C₅ alkylthio, C₁-C₅ alkane sulfinyl, C₁-C₅ alkane sulfonyl, a substituted phenyl, a substituted phenoxy, a substituted phenylthio, a substituted phenylene sulfinyl, or a substituted benzene sulfonyl; and n₁, n₂ at each occurrence independently are an integer from 1 to 5.

9. The method of claim 8, wherein the compound represented by formula 5 is dissolved in a solvent and reacts with an anhydride or acyl chloride in the presence of a base to yield the compound represented by formula 6.

10. The method of claim 9, wherein the base is triethylamine, pyridine, dimethylaminopyridine, diisopropylethylamine, sodium carbonate, potassium carbonate, sodium bicarbonate, or a mixture thereof; the solvent is dichloromethane, chloroform, tetrahydrofuran, acetone, and ethyl acetate, or a mixture thereof.

11. The method of claim 9, wherein a molar ratio of the compound represented by formula 5 to the acyl chloride or anhydride to the base is 1: (0.8-2): (0.8-2).

12. The method of claim 8, wherein the compound represented by formula 6 is prepared by the compound represented by formula 5 under a temperature of 0-40°C.

13. The method of claim 8, wherein the compound represented by formula 5 is prepared by a compound represented by formula 4:

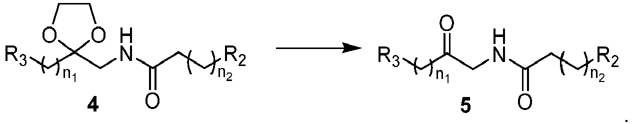

14. The method of claim 13, wherein the compound represented by formula 4 is dissolved in a solvent and converted into the compound represented by formula 5 through a deprotection reaction in the presence of an acidity regulator.

15. The method of claim 14, wherein the acidity regulator is hydrochloric acid, sulfuric acid, formic acid, acetic acid, or a mixture thereof; and the solvent is dichloromethane, chloroform, tetrahydrofuran, acetone, and ethyl acetate, or a mixture thereof.

16. The method of claim 15, wherein a molar ratio of the compound represented by formula 4 to the acidity regulator is between 1: 0.1 and 1: 1.

17. The method of claim 13, wherein the compound represented by formula 5 is prepared by the compound represented by formula 4 under a temperature of 20-30 °C.

18. The method of claim 13, wherein the compound represented by formula 4 is prepared by reaction of a compound represented by formula 2 and a compound represented by formula 3 as follows:

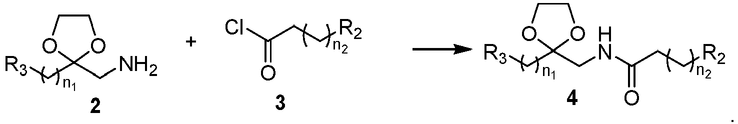

19. The method of claim 18, wherein the compound represented by formula 2 and the compound represented by formula 3 are dissolved in a solvent and react with each other in the presence of a base to yield the compound represented by formula 4.

20. The method of claim 19, wherein the solvent is dichloromethane, chloroform, tetrahydrofuran, acetone, and ethyl acetate, or a mixture thereof, and the base is triethylamine, pyridine, dimethylaminopyridine, diisopropylethylamine, sodium carbonate, potassium carbonate, sodium bicarbonate, or a mixture thereof.

21. The method of claim 19, wherein a molar ratio of the compound represented by formula 2 to the compound represented by formula 3 to the base is 1: (0.8-2): (0.8-2).

22. The method of claim 18, wherein the compound represented by formula 4 is prepared by reaction of the compound represented by formula 2 and the compound represented by formula 3 under a temperature of 15-30°C.
